# EUROPEAN PATENT APPLICATION

(11) **EP 4 582 087 A1**
(43) Date of publication of application: **09.07.2025**
(21) Application number: 23860419.3
(22) Date of filing: 30.08.2023
(51) Int. Cl.: A61K 31/675, A61K 45/00, A61P 35/00, A61P 43/00

(54) **PHARMACEUTICAL COMPOSITION FOR TREATING LYMPHOMA**

(30) Priority: 31.08.2022 WO PCT/JP2022/032837; 02.11.2022 JP 2022176670
(71) Applicant: SymBio Pharmaceuticals Limited, Tokyo 105-0001 (JP)
(72) Inventor: CHAN, Jason, 822312 (SG); ONG, Choon Kiat, 510146 (SG); HAZAMA, Masatoshi, Tokyo 105-0001 (JP); FUKUSHIMA, Koji, Tokyo 105-0001 (JP)
(74) Representative: Gulde & Partner
(86) International application number: PCT/JP2023/031556
(87) International publication number: WO 2024/048657

(57) **Abstract**

A method for treating lymphoma is provided. A pharmaceutical composition for treatment of lymphoma, including BCV, a pharmaceutically acceptable salt thereof, or a solvate thereof is used. The lymphoma may be EBV-positive lymphoma. The lymphoma may be MYC-positive lymphoma. The pharmaceutical composition may be used in combination with a chemotherapeutic agent.

## Description

### Technical Field

The technical field of the present invention relates to treatment of lymphoma.

### Background art

Lymphoma, a type of blood cancer, is known as a disease caused by cancerous changes in lymphocytes. Non-Patent Literature 1 states that non-Hodgkin's lymphoma is the most common hematological malignancy in the world, accounting for nearly 3% of cancer diagnoses and cancer deaths. Non-Patent Literature 2 states that the incidence rate of Hodgkin's lymphoma has been rising over the past ten years, especially among females, the younger population, and people from Asian countries.

Several reports on lymphoma treatment methods have been published in recent years. Non-Patent Literature 3 describes the potential of a treatment that targets CD19. Non-Patent Literature 4 describes the potential of a treatment that targets EZH2.

### Citation List

### Non-Patent Literature

[Non Patent Literature 1]
   "Epidemiology of Non-Hodgkin's Lymphoma" Thandra et al., Med Sci (Basel). 2021 Jan 30; 9(1): 5.
[Non Patent Literature 2]
   "Incidence, mortality, risk factors, and trends for Hodgkin lymphoma: a global data analysis" Huang et al., J Hematol Oncol. 2022 May 11; 15(1): 57.
[Non Patent Literature 3]
   "Targeting CD19 for diffuse large B cell lymphoma in the era of CARs: Other modes of transportation" Blood Rev. 2022 Aug 17; 101002.
[Non Patent Literature 4]
   "Taking the EZ way: Targeting enhancer of zeste homolog 2 in B-cell lymphomas" Morschhauser et al., Blood Rev. 2022 Jul 9; 100988.

### Summary of Invention

### Technical Problem

Despite various findings accumulated thus far, the mechanism of lymphoma is still unknown in many respects, and thus, conventional therapeutic approaches alone have not been satisfactorily successful.

### Solution to Problem

In contrast, the present inventors conducted intensive research and found that brincidofovir (hereinafter referred to as BCV) exhibits a good growth-suppressive effect on lymphoma cells.

Specifically, one aspect of the present invention provides a pharmaceutical composition for treatment of lymphoma, comprising BCV, a pharmaceutically acceptable salt thereof, or a solvate thereof. This pharmaceutical composition can be used to treat lymphoma.

### Brief Description of Drawings

[Figure 1] Figs. 1A to 1C show the results of an examination regarding the effect of BCV treatment on the proliferation of lymphoma cells. Figs. 1A and 1B show the results for EBV-positive cells, and Fig. 1C shows the results for EBV-negative cells.
[Figure 2] Fig. 2 shows the results of an examination regarding the effect of treatment with BCV or various antiviral agents on the proliferation of EBV-positive NK/T lymphoma cells.
[Figure 3] Figs. 3A to 3D show the results of administration of BCV to an NSG mouse implanted with lymphoma cells. Fig. 3A shows tumor sizes measured on days 1, 5, 8, 12, and 15. Fig. 3B shows body weights measured on days 1, 5, 8, 12, and 15. Fig. 3C shows the measurement results of tumor weights. In the bar graph, the left bar represents a vehicle-receiving group and the right bar represents a BCV-receiving group. Fig. 3D shows a photograph of tumors collected from the respective individuals.
[Figure 4] Fig. 4 shows the results of an examination regarding the effect of BCV treatment on the expression of the EBNA1 and LMP1 genes in lymphoma cells.
[Figure 5] Fig. 5A shows the results of an examination regarding the effect of BCV treatment on the expression of the Myc gene in lymphoma cells. In the bar graphs of Figs. 5A to 5C, the values are presented in groups of three bars. Within each group, the bars, from left to right, correspond to DMSO, BCV 0.1 µg/mL (0.17 µM), and BCV 1 µg/mL (1.7 µM), respectively. Figs. 5B and 5C show the results of an examination regarding the effect of BCV treatment on the expression of the gene group associated with the STING pathway in lymphoma cells.
[Figure 6] Fig. 6 shows the results of an examination regarding the effect of BCV treatment on immunogenic cell death. In the bar graph of Fig. 6, the values are presented in groups of three bars. Within each group, the bars, from left to right, correspond to DMSO, BCV 0.1 µg/mL (0.17 µM), and BCV 1 µg/mL (1.7 µM), respectively.
[Figure 7] Figs. 7A to 7C show the results of an examination regarding the effect of treatment with BCV and each anticancer agent or treatment with BCV and irradiation, on the proliferation of lymphoma cells. Fig. 7A shows the results of treatment with etoposide or gemcitabine. Fig. 7B shows the results of treatment with BCV alone, BCV and etoposide, or BCV and gemcitabine. Fig. 7C shows the results of treatment with BCV and irradiation.
[Figure 8] Fig. 8 shows the results of an examination regarding the effect of BCV treatment on the proliferation of MYC-amplified lymphoma (EBV-positive or EBV-negative).
[Figure 9] Fig. 9 shows the results of an examination regarding the sensitivity of eleven lymphoma cell lines to the effect of BCV treatment on proliferation.
[Figure 10] Figs. 10 A and 10B show the results of gene expression analysis. Fig. 10A shows the gene sets that exhibited increased expression in BCV-sensitive cell lines. Fig. 10B shows the enrichment plot of MYC TARGETS V2.
[Figure 11] Figs. 11A and 11B show the results of an examination regarding the prognosis of a human patient cohort. Fig. 11A shows the results of an examination regarding progression-free survival. Fig. 11B shows the results of an examination regarding overall survival.
[Figure 12] Fig. 12 shows the results of an examination regarding genes that exhibited increased expression in the poor-prognosis group of the human patient cohort.

### Description of Embodiments

Hereinafter, embodiments of the present invention will be described in detail. Note that repeated descriptions of similar content are omitted where appropriate in order to avoid redundancy.

### (1) Treatment method

An embodiment of the present invention provides a method for treating lymphoma, comprising the step of administering BCV, a pharmaceutically acceptable salt thereof, or a solvate thereof to a subject. This treatment method can be used to treat lymphoma through a novel therapeutic approach.

The above-described lymphoma may be MYC-positive lymphoma. In this case, BCV, a pharmaceutically acceptable salt thereof, or a solvate thereof may exert a particularly good therapeutic effect. For example, for MYC-positive lymphoma, a strong growth-suppressive effect may be achieved not only in EBV-positive lymphoma but also in EBV-negative lymphoma.

In the treatment method described above, the subject may be, for example, an MYC-positive subject. In this case, BCV, a pharmaceutically acceptable salt thereof, or a solvate thereof may exert a particularly good therapeutic effect. For example, for MYC-positive subjects, a strong growth-suppressive effect may be achieved not only in a subject suffering from EBV-positive lymphoma but also in a subject suffering from EBV-negative lymphoma.

The treatment method described above may comprise, for example, a step of identifying an MYC-positive subject. In this case, BCV, a pharmaceutically acceptable salt thereof, or a solvate thereof may exert a particularly good therapeutic effect. For example, for MYC-positive subjects, a strong growth-suppressive effect may be achieved not only in a subject suffering from EBV-positive lymphoma but also in a subject suffering from EBV-negative lymphoma.

In the treatment method described above, the subject may be, for example, a subject identified based on the expression level of MYC as an index. In this case, BCV, a pharmaceutically acceptable salt thereof, or a solvate thereof may exert a particularly good therapeutic effect. For example, a stronger growth-suppressive effect on lymphoma may be achieved by identifying and treating a subject with a high MYC expression level, compared to treating a subject with a low MYC expression level.

The treatment method described above may comprise, for example, a step of identifying a subject with high MYC expression as a subject to be treated. In this case, BCV, a pharmaceutically acceptable salt thereof, or a solvate thereof may exert a particularly good therapeutic effect. For example, a stronger growth-suppressive effect on lymphoma may be achieved by treating a subject with high MYC expression, compared to treating a subject without high MYC expression.

The treatment method described above may comprise, for example, a step of detecting the MYC expression level in a sample from a subject. In this case, BCV, a pharmaceutically acceptable salt thereof, or a solvate thereof may exert a particularly good therapeutic effect. For example, a strong therapeutic effect may be achieved by treating a subject whose sample showed a high MYC expression level. For example, when the MYC expression level in a sample from a subject is high, the subject may be identified as a subject for BCV administration or may be identified as a subject for BCV administration at a low dose. On the other hand, for example, when the MYC expression level in a sample from a subject is low, the subject may be identified as a subject for no BCV administration or may be identified as a subject for BCV administration at a high dose.

The treatment method described above may comprise, for example, a step of administering BCV, a pharmaceutically acceptable salt thereof, or a solvate thereof, to a subject whose sample showed a high MYC expression level, at a higher dose compared to a subject whose sample showed a low MYC expression level. In this case, BCV, a pharmaceutically acceptable salt thereof, or a solvate thereof may exert a particularly good therapeutic effect.

The treatment method described above comprises, for example, a step of administering brincidofovir, a pharmaceutically acceptable salt thereof, or a solvate thereof to a subject, wherein the subject may be a subject whose sample was demonstrated to have a higher MYC expression level compared to the MYC expression level of a sample previously confirmed not to have high MYC expression, by detecting the MYC expression level in the sample from the subject. In this case, BCV, a pharmaceutically acceptable salt thereof, or a solvate thereof may exert a particularly good therapeutic effect.

The treatment method described above may comprise, for example, a step of administering brincidofovir, a pharmaceutically acceptable salt thereof, or a solvate thereof to a subject, wherein the subject is a subject whose sample was demonstrated to have a higher MYC expression level compared to the MYC expression level of an MYC-negative sample, by detecting the MYC expression level in the sample from the subject. In this case, BCV, a pharmaceutically acceptable salt thereof, or a solvate thereof may exert a particularly good therapeutic effect.

The lymphoma described above may be EBV-positive lymphoma. In this case, BCV, a pharmaceutically acceptable salt thereof, or a solvate thereof may exert a particularly good therapeutic effect. For example, a stronger growth-suppressive effect may be achieved in the EBV-positive lymphoma compared to EBV-negative lymphoma.

In the treatment method described above, the subject may be, for example, an EBV-positive subject. In this case, BCV, a pharmaceutically acceptable salt thereof, or a solvate thereof may exert a particularly good therapeutic effect. For example, a stronger growth-suppressive effect may be achieved in an EBV-positive subject compared to a subject with EBV-negative lymphoma.

The treatment method described above may comprise, for example, a step of identifying an EBV-positive subject. In this case, BCV, a pharmaceutically acceptable salt thereof, or a solvate thereof may exert a particularly good therapeutic effect. For example, a stronger growth-suppressive effect may be achieved in an EBV-positive subject compared to a subject with EBV-negative lymphoma.

In the treatment method described above, the subject may be, for example, a subject identified based on the expression level of EBV as an index. In this case, BCV, a pharmaceutically acceptable salt thereof, or a solvate thereof may exert a particularly good therapeutic effect. For example, a stronger growth-suppressive effect on lymphoma may be achieved by identifying and treating a subject with a high EBV expression level, compared to treating a subject with a low EBV expression level.

The treatment method described above may comprise, for example, a step of identifying a subject with high EBV expression as a subject to be treated. In this case, BCV, a pharmaceutically acceptable salt thereof, or a solvate thereof may exert a particularly good therapeutic effect. For example, a stronger growth-suppressive effect on lymphoma may be achieved by treating a subject with high EBV expression, compared to treating a subject without high EBV expression.

The treatment method described above may comprise, for example, a step of detecting the EBV expression level in a sample from a subject. In this case, BCV, a pharmaceutically acceptable salt thereof, or a solvate thereof may exert a particularly good therapeutic effect. For example, a strong therapeutic effect may be achieved by treating a subject whose sample showed a high EBV expression level. For example, when the EBV expression level in a sample from a subject is high, the subject may be identified as a subject for BCV administration or may be identified as a subject for BCV administration at a low dose. On the other hand, for example, when the EBV expression level in a sample from a subject is low, the subject may be identified as a subject for no BCV administration or may be identified as a subject for BCV administration at a high dose.

The treatment method described above may comprise, for example, a step of administering BCV, a pharmaceutically acceptable salt thereof, or a solvate thereof, to a subject whose sample showed a high EBV expression level, at a higher dose compared to a subject whose sample showed a low EBV expression level. In this case, BCV, a pharmaceutically acceptable salt thereof, or a solvate thereof may exert a particularly good therapeutic effect.

The treatment method described above comprises, for example, a step of administering brincidofovir, a pharmaceutically acceptable salt thereof, or a solvate thereof to a subject, wherein the subject may be a subject whose sample was demonstrated to have a higher EBV expression level compared to the EBV expression level of a sample previously confirmed not to have high EBV expression, by detecting the EBV expression level in the sample from the subject. In this case, BCV, a pharmaceutically acceptable salt thereof, or a solvate thereof may exert a particularly good therapeutic effect.

The treatment method described above may comprise, for example, a step of administering brincidofovir, a pharmaceutically acceptable salt thereof, or a solvate thereof to a subject, wherein the subject is a subject whose sample was demonstrated to have a higher EBV expression level compared to the EBV expression level of an EBV-negative sample, by detecting the EBV expression level in the sample from the subject. In this case, BCV, a pharmaceutically acceptable salt thereof, or a solvate thereof may exert a particularly good therapeutic effect.

The treatment method described above may comprise, for example, a step of identifying an MYC-positive and EBV-positive subject as a subject to be treated, a step of identifying a subject based on the expression level of MYC and EBV as an index, or a step of identifying a subject with high expression of MYC and EBV as a subject to be treated. In this case, BCV, a pharmaceutically acceptable salt thereof, or a solvate thereof may exert a particularly good therapeutic effect. The lymphoma described above may be MYC-positive and EBV-positive lymphoma. In this case, BCV, a pharmaceutically acceptable salt thereof, or a solvate thereof may exert a particularly good therapeutic effect. The treatment method described above may comprise, for example, a step of identifying an MYC-positive and EBV-negative subject. The lymphoma described above may be MYC-positive and EBV-negative lymphoma.

The lymphoma described above may be, for example, EBV-positive NK/T lymphoma, MYC-positive EBV-positive NK/T lymphoma, MYC-positive Burkitt's lymphoma, MYC-positive DLBCL, MYC-positive double-hit DLBCL, or MYC-positive triple-hit DLBCL. In this case, BCV, a pharmaceutically acceptable salt thereof, or a solvate thereof may exert a particularly good therapeutic effect.

The treatment method described above may comprise a step of administering a chemotherapeutic agent to a subject. In this case, the combined use leads to a particularly good therapeutic effect. For example, a synergistic effect of BCV and the chemotherapeutic agent is achieved by such combination treatment. Examples of the synergistic effect include an effect greater than the sum of the growth-suppressive effect on lymphoma obtained by monotherapy with BCV and the growth-suppressive effect on lymphoma obtained by monotherapy with the chemotherapeutic agent. A particularly good therapeutic effect may be achieved by using an antimetabolite or a topoisomerase inhibitor in combination with BCV. A particularly good therapeutic effect may also be achieved by using an immune checkpoint inhibitor in combination with BCV. The chemotherapeutic agent may be administered to the subject prior to, concurrently with, or subsequent to BCV administration. Concurrent administration includes administration within the same time period and any administration performed substantially concurrently, taking usual treatment procedures into account. Concurrent administration includes administration of BCV and a chemotherapeutic agent as a combination preparation.

The treatment method described above may comprise a step of performing irradiation on a subject. Examples of irradiation include gamma irradiation. The irradiation may be performed on the subject prior to, concurrently with, or subsequent to BCV administration.

The treatment method described above may, for example, reduce MYC expression in lymphoma cells in a subject by administering BCV, a pharmaceutically acceptable salt thereof, or a solvate thereof to the subject. This treatment method can improve prognosis of a human lymphoma patient by reducing MYC expression.

The treatment method described above may, for example, induce immunogenic cell death by administering BCV, a pharmaceutically acceptable salt thereof, or a solvate thereof to a subject.

Examples of the subject (including a patient) in the treatment method described above include a human or a non-human mammal (e.g., at least one species selected from a mouse, a guinea pig, a hamster, a rat, a mouse, a rabbit, a pig, a sheep, a goat, a cow, a horse, a cat, a dog, a marmoset, a monkey, or a chimpanzee). The patient may be a patient diagnosed as having lymphoma (e.g., MYC-positive or EBV-positive lymphoma) or a patient in need of treatment of lymphoma. The patient may also be, for example, a patient to whom a chemotherapeutic agent was administered; a patient who is receiving treatment with a chemotherapeutic agent; a patient to whom BCV, a pharmaceutically acceptable salt thereof, or a solvate thereof was administered; or a patient who is receiving treatment with BCV, a pharmaceutically acceptable salt thereof, or a solvate thereof. Preferably, the subject for the treatment with BCV, a pharmaceutically acceptable salt thereof, or a solvate thereof is a patient having NK/T-cell lymphoma. In this case, in particular, effects such as a good therapeutic effect on NK/T-cell lymphoma, an effect of minimizing side effects, an effect of reducing MYC expression, or an effect of improving prognosis may be achieved. Here, it is preferable that the NK/T-cell lymphoma be EBV-positive or exhibit high EBV expression. In this case, a particularly good therapeutic effect on NK/T-cell lymphoma may be achieved. Furthermore, it is preferable that the NK/T-cell lymphoma be MYC-positive or exhibit high MYC expression. In this case, a particularly good therapeutic effect on NK/T-cell lymphoma may be achieved.

The treatment method described above may comprise, for example, (i) a step of identifying an MYC-positive or EBV-positive subject as a subject to be treated for lymphoma, (ii) a step of identifying a subject having MYC-positive lymphoma or EBV-positive lymphoma as a subject to be treated for lymphoma, (iii) a step of detecting MYC or EBV in a subject, (iv) a step of detecting whether a subject is MYC-positive or EBV-positive, (v) a step of collecting a sample from a subject (e.g., a lymphoma cell, lymph node tissue, or a blood sample such as plasma, serum, or whole blood), (vi) a step of detecting MYC or EBV in a collected sample, or (vii) a step of identifying, as a subject to be treated, a subject from whom a sample was collected, wherein MYC or EBV was detected in the sample. Comprising one or more of these steps is useful for treating a population in which the treatment is highly effective. When two or more of these steps are comprised, their order is arbitrary and can be determined according to the desired treatment method. These steps may be performed prior to or subsequent to the step of administering BCV, a pharmaceutically acceptable salt thereof, or a solvate thereof, or a chemotherapeutic agent to a subject. The detection may be based on the amount of gene, mRNA, or protein, or on gene translocation as an index. The detection may be a test.

The treatment method described above may comprise, for example, a step of identifying a subject having lymphoma as a subject to be treated for lymphoma; a step of identifying a subject who is positive for a lymphoma marker as a subject to be treated for lymphoma; a step of administering a therapeutically effective amount of BCV or a chemotherapeutic agent to a subject; a step of suppressing the proliferation of lymphoma cells in a subject; a step of reducing a lymphoma marker in a subject; a step of reducing MYC expression in the lymphoma of a subject; or a step of suppressing swelling of the lymph node of a subject. The treatment method or subject described above optionally may or may not comprise, for example: a step of identifying a subject infected with cytomegalovirus (CMV), adenovirus (AdV), BK virus (BKV), or variola virus (VaV); a step of identifying a subject seropositive for CMV, AdV, BKV, or VaV; a step of identifying a subject in need of prevention or treatment of infection with CMV, AdV, BKV, or VaV; a step of identifying a subject receiving allogeneic transplantation or after receiving allogeneic transplantation (e.g., hematopoietic cell transplantation); a step of identifying a subject in need of an immunosuppressive agent; a step of identifying a subject in an immunosuppressed state; a method for treating a virus-induced tumor in a subject in an immunosuppressed state; or a method for preventing or treating infection with a virus (e.g., CMV, AdV, BKV, or VaV).

### (2) Pharmaceutical composition

An embodiment of the present invention provides a pharmaceutical composition for treatment of lymphoma, comprising BCV, a pharmaceutically acceptable salt thereof, or a solvate thereof. This pharmaceutical composition can be used to treat lymphoma through a novel therapeutic approach.

The lymphoma described above may be MYC-positive lymphoma. **In** this case, BCV, a pharmaceutically acceptable salt thereof, or a solvate thereof may exert a particularly good therapeutic effect. For example, for MYC-positive lymphoma, a strong growth-suppressive effect may be achieved not only in EBV-positive lymphoma but also in EBV-negative lymphoma.

The lymphoma described above may be EBV-positive lymphoma. **In** this case, BCV, a pharmaceutically acceptable salt thereof, or a solvate thereof may exert a particularly good therapeutic effect. For example, a stronger growth-suppressive effect may be achieved in EBV-positive lymphoma compared to EBV-negative lymphoma.

In the treatment described above, the subject may be, for example, a subject identified based on the expression level of EBV or MYC as an index. In this case, BCV, a pharmaceutically acceptable salt thereof, or a solvate thereof may exert a particularly good therapeutic effect. For example, a stronger growth-suppressive effect on lymphoma may be achieved by identifying and treating a subject with a high EBV or MYC expression level, compared to treating a subject with a low EBV or MYC expression level.

The treatment described above may comprise, for example, detecting the EBV or MYC expression level in a sample from a subject. In this case, BCV, a pharmaceutically acceptable salt thereof, or a solvate thereof may exert a particularly good therapeutic effect. For example, a strong therapeutic effect may be achieved by treating a subject whose sample showed a high EBV or MYC expression level.

The treatment described above comprises, for example, administering brincidofovir, a pharmaceutically acceptable salt thereof, or a solvate thereof to a subject, wherein the subject may be a subject whose sample was demonstrated to have a higher EBV or MYC expression level compared to the EBV or MYC expression level of a sample previously confirmed not to have high EBV or MYC expression, by detecting the EBV or MYC expression level in the sample from the subject. In this case, BCV, a pharmaceutically acceptable salt thereof, or a solvate thereof may exert a particularly good therapeutic effect.

The lymphoma described above may be MYC-positive and EBV-positive lymphoma. In this case, BCV, a pharmaceutically acceptable salt thereof, or a solvate thereof may exert a particularly good therapeutic effect.

The lymphoma described above may be, for example, EBV-positive NK/T lymphoma, MYC-positive EBV-positive NK/T lymphoma, MYC-positive Burkitt's lymphoma, MYC-positive DLBCL, MYC-positive double-hit DLBCL, or MYC-positive triple-hit DLBCL. In this case, BCV, a pharmaceutically acceptable salt thereof, or a solvate thereof may exert a particularly good therapeutic effect.

The pharmaceutical composition described above may be used in combination treatment of BCV, a pharmaceutically acceptable salt thereof, or a solvate thereof and a chemotherapeutic agent. In this case, the combined use leads to a particularly good therapeutic effect. For example, a synergistic effect of BCV and the chemotherapeutic agent is achieved by such combination treatment. Examples of the synergistic effect include an effect greater than the sum of the growth-suppressive effect on lymphoma obtained by monotherapy with BCV and the growth-suppressive effect on lymphoma obtained by monotherapy with the chemotherapeutic agent.

The pharmaceutical composition described above includes a pharmaceutical composition comprising BCV, a pharmaceutically acceptable salt thereof, or a solvate thereof for use in the treatment of lymphoma. Another aspect of the present invention provides a use of BCV, a pharmaceutically acceptable salt thereof, or a solvate thereof for the manufacture of a pharmaceutical composition for treatment of lymphoma.

An embodiment of the present invention provides a pharmaceutical composition for treatment of lymphoma, comprising BCV, a pharmaceutically acceptable salt thereof, or a solvate thereof, or a chemotherapeutic agent, wherein the pharmaceutical composition is for use in the combination treatment of BCV, a pharmaceutically acceptable salt thereof, or a solvate thereof and a chemotherapeutic agent. The use of this pharmaceutical composition leads to a particularly good therapeutic effect due to combined use, as described above.

The pharmaceutical composition described above includes a pharmaceutical composition for use in the treatment method described in (1) above. The treatment method may comprise at least one of the steps described in (1) above (for example, including steps (i) to (vii)).

### (3) Inhibition method

An embodiment of the present invention provides a method for inhibiting MYC, comprising the step of administering BCV, a pharmaceutically acceptable salt thereof, or a solvate thereof to a subject. The proliferation of MYC-positive lymphoma cells is suppressed by inhibiting MYC. Thus, this inhibition method can be used to suppress the proliferation of MYC-positive lymphoma cells more effectively. This method may comprise at least one of the steps described in (1) above (for example, including steps (i) to (vii)). This method includes a method for treating a disease by inhibiting MYC. Examples of diseases include a disease caused by MYC. Examples of diseases include lymphoma (e.g., MYC-positive lymphoma).

An embodiment of the present invention provides a composition for inhibition of MYC, the composition comprising BCV, a pharmaceutically acceptable salt thereof, or a solvate thereof. The proliferation of MYC-positive lymphoma cells is suppressed by inhibiting MYC. Thus, this composition can be used to suppress the proliferation of MYC-positive lymphoma cells more effectively. This composition includes a composition for use in the inhibition method described above.

The composition described above includes a composition comprising BCV, a pharmaceutically acceptable salt thereof, or a solvate thereof for use in the inhibition of MYC. Another aspect of the present invention provides a use of BCV, a pharmaceutically acceptable salt thereof, or a solvate thereof for the manufacture of a composition for inhibition of MYC.

An embodiment of the present invention provides a method for inhibiting MYC, comprising the step of contacting MYC with BCV, a pharmaceutically acceptable salt thereof, or a solvate thereof. Examples of inhibition include in vitro, ex vivo, or in vivo inhibition.

An embodiment of the present invention provides a method for inhibiting EBNA1 or LMP1, comprising the step of administering BCV, a pharmaceutically acceptable salt thereof, or a solvate thereof to a subject. This inhibition method can be used to suppress the proliferation of EBV-positive lymphoma cells more effectively. This method may comprise at least one of the steps described in (1) above (for example, including steps (i) to (vii)). This method includes a method for treating a disease by inhibiting EBNA1 or LMP1. Examples of diseases include a disease caused by EBV. Examples of the disease include lymphoma (e.g., EBV-positive lymphoma). Another aspect of the present invention provides a composition for inhibition of EBNA1 or LMP1, the composition comprising BCV, a pharmaceutically acceptable salt thereof, or a solvate thereof. Another aspect of the present invention provides a use of BCV, a pharmaceutically acceptable salt thereof, or a solvate thereof for the manufacture of a composition for inhibition of EBNA1 or LMP1. Another aspect of the present invention provides a method for inhibiting EBNA1 or LMP1, comprising the step of contacting EBNA1 or LMP1 with BCV, a pharmaceutically acceptable salt thereof, or a solvate thereof. Examples of inhibition include in vitro, ex vivo, or in vivo inhibition.

### (4) Diagnostic method

An embodiment of the present invention provides a companion diagnostic method, comprising the step of detecting whether a subject is MYC-positive or not. This method includes a diagnostic method for evaluating, prior to administration, the efficacy of treatment with BCV, a pharmaceutically acceptable salt thereof, or a solvate thereof. This diagnostic method allows for treating a population in which the treatment is highly effective. Here, the subject includes a patient suffering from lymphoma or the patient's lymphoma.

An embodiment of the present invention provides a companion diagnostic method, comprising the step of detecting whether a subject is EBV-positive or not. This method includes a diagnostic method for evaluating, prior to administration, the efficacy of treatment with BCV, a pharmaceutically acceptable salt thereof, or a solvate thereof. This diagnostic method allows for treating a population in which the treatment is highly effective. Here, the subject includes a patient suffering from lymphoma or the patient's lymphoma.

An embodiment of the present invention provides a companion diagnostic method, comprising the step of detecting whether a subject is MYC-positive and EBV-positive or not. This method includes a diagnostic method for evaluating, prior to administration, the efficacy of treatment with BCV, a pharmaceutically acceptable salt thereof, or a solvate thereof. This diagnostic method allows for treating a population in which the treatment is highly effective. The subject in the diagnostic method includes a patient suffering from lymphoma or the patient's lymphoma.

An embodiment of the present invention provides a companion diagnostic method, comprising the step of detecting EBV expression or MYC expression in a subject. This method includes a diagnostic method for evaluating, prior to administration, the efficacy of treatment with BCV, a pharmaceutically acceptable salt thereof, or a solvate thereof. This diagnostic method allows for treating a population in which the treatment is highly effective. Here, the subject includes a patient suffering from lymphoma or the patient's lymphoma.

An embodiment of the present invention provides a method for diagnosing whether a subject has lymphoma that is highly sensitive to BCV, a pharmaceutically acceptable salt thereof, or a solvate thereof, comprising the step of examining the expression level of EBV or MYC in a sample from the subject having lymphoma. This method can be used to diagnose whether a subject has lymphoma that is highly sensitive to BCV, based on the expression level of EBV or MYC as an index. Thus, more appropriate BCV treatment can be performed by using this method. For example, when a subject is diagnosed as having highly sensitive lymphoma, treatment may be performed by administering BCV or by administering BCV at a low dose. On the other hand, for example, when a subject is diagnosed as not having highly sensitive lymphoma, BCV is not administered or treatment may be performed by administering BCV at a high dose. This diagnosis may be a companion diagnosis for treatment with BCV, a pharmaceutically acceptable salt thereof, or a solvate thereof.

In another aspect, an embodiment of the present invention provides a kit or a composition for any of the diagnostic methods or companion diagnostic methods described above. The kit or the composition includes, for example, a means for measuring the amount of gene, mRNA, or protein, or gene translocation of MYC or EBV. Examples of the measuring means may include a primer (e.g., a primer capable of amplifying at least a part of the MYC or EBV gene, such as a primer that can bind to the MYC or EBV gene or to an upstream or downstream site thereof) or a probe (a probe capable of detecting MYC gene translocation, such as a FISH probe).

### (5) Other methods

An embodiment of the present invention provides a method for identifying a subject to be treated with BCV, a pharmaceutically acceptable salt thereof, or a solvate thereof, comprising the step of detecting MYC or EBV in the subject. This method allows for treating a population in which the treatment is highly effective. For example, when the EBV or MYC expression level in the subject is high and the subject is expected to have high sensitivity, the subject may be identified as a subject for BCV administration or as a subject for BCV administration at a low dose. On the other hand, for example, when the EBV or MYC expression level in the subject is low and the subject is expected to have low sensitivity, the subject may be identified as a subject for no BCV administration or as a subject for BCV administration at a high dose. Here, examples of the subject include a patient suffering from lymphoma or a sample from the patient.

An embodiment of the present invention provides a method for improving the prognosis of a subject in lymphoma treatment, comprising the step of administering BCV, a pharmaceutically acceptable salt thereof, or a solvate thereof to the subject. BCV, a pharmaceutically acceptable salt thereof, or a solvate thereof has the effect of reducing MYC expression, and thus, may improve the prognosis of a subject in lymphoma treatment. Examples of the prognosis improvement include achieving a more favorable prognosis. Examples of the prognosis improvement include the extension of progression-free survival or survival time. Examples of the prognosis improvement include a state in which the prognosis of a subject is improved compared to that of a patient who has undergone a treatment method that does not reduce MYC expression. Examples of prognosis include any change in a patient's symptoms over the course of a disease.

An embodiment of the present invention provides a method for predicting or evaluating the prognosis of a malignant tumor, comprising the step of using the MYC expression level in a subject having lymphoma as an index. This method can be used to predict or evaluate the prognosis of a patient. For example, when the MYC expression level in a sample from a subject having lymphoma is high, the prognosis may be predicted or evaluated to be poor (poor prognosis). For example, when the MYC expression level in a sample from a subject having lymphoma is low, the prognosis may be predicted or evaluated to be favorable (favorable prognosis).

The embodiments described in (1) to (5) above will be described in more detail below. The following detailed embodiments or descriptions of terms are applicable to all the embodiments described in (1) to (5) above.

In (1) to (5) above, BCV (brincidofovir) includes a compound having a structure represented by the following formula. BCV may also be represented by the IUPAC name: [(2S)-1-(4-amino-2-oxopyrimidin-1-yl)-3-hydroxypropan-2-yl]oxymethyl-(3-hexadecoxypropoxy)phosphinic acid or ({[(2S)-1-(4-amino-2-oxo-1,2-dihydropyrimidin-1-yl)-3-hydroxypropan-2-yl]oxy}methyl)[3-(hexadecyloxy)propoxy]phosphinic acid. BCV includes a compound represented by CAS Registry Number 444805-28-1. In the present specification, BCV is an abbreviation for brincidofovir, and both have the same meaning.

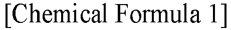

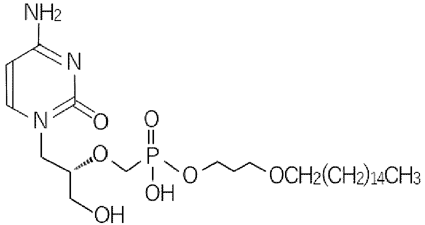

In (1) to (5) above, examples of lymphomas include malignant lymphoma. Examples of malignant lymphomas include a disease caused by cancerous changes in lymphocytes. Examples of malignant lymphomas include non-Hodgkin's lymphoma or Hodgkin's lymphoma. Examples of non-Hodgkin's lymphomas include B-cell lymphoma or NK/T-cell lymphoma. Examples of B-cell lymphomas include follicular lymphoma, MALT lymphoma, lymphoplasmacytic lymphoma, mantle cell lymphoma, diffuse large B-cell lymphoma (DLBCL), Burkitt's lymphoma, primary effusion lymphoma, or chronic lymphocytic leukemia/small lymphocytic lymphoma. Examples of NK/T-cell lymphoma include peripheral T-cell lymphoma, angioimmunoblastic T-cell lymphoma, anaplastic large cell lymphoma, adult T-cell leukemia-lymphoma, extranodal NK/T-cell lymphoma, nasal type, or cutaneous lymphoma (e.g., mycosis fungoides). Examples of Hodgkin's lymphomas include classical Hodgkin's lymphoma or nodular lymphocyte predominant Hodgkin's lymphoma. For details of lymphoma, reference may be made to, for example, Nirmal, J Oral Maxillofac Pathol. 2020 May-Aug; 24(2): 195-199, Singh et al., J Family Med Prim Care. 2020 Apr; 9(4): 1834-1840, or Voltin et al., Cancers (Basel). 2020 Mar 5; 12(3): 601. The therapeutic effect on lymphoma or the onset of lymphoma may be determined by examining lymph node swelling with CT, PET, or MRI, by performing a histological examination of a lymph node or a lump, or by analyzing a tumor marker of malignant lymphoma in blood (e.g., sIL2-R). The diagnosis of lymphoma may be carried out using, for example, the method described in Nirmal (supra) or Voltin et al. (supra). The therapeutic effect on lymphoma may be evaluated, for example, by observing how the proliferation of lymphoma cells changes over time after drug administration. A state in which cell proliferation is suppressed includes a state in which the proliferation rate of the test cells is significantly decreased compared to that before drug treatment. The proliferation rate may be measured, for example, using absorbance as an index or may be determined based on image data. The therapeutic effect on lymphoma may be evaluated, for example, by observing a reduction in tumor mass after drug administration. When the tumor mass is significantly reduced compared to that before drug administration or after negative control administration, a therapeutic effect may be considered present. The therapeutic effect on lymphoma may be measured, for example, using the amount of a lymphoma marker in a patient or a patient-derived sample as an index. When the maker amount is significantly reduced compared to that before drug administration or after negative control administration, a therapeutic effect may be considered present. The tumor mass or marker amount after drug administration may be reduced to 0.9, 0.7, 0.5, 0.3, or 0.1 times or less compared to that before drug administration or after negative control administration.

In (1) to (5) above, the lymphoma includes MYC-positive lymphoma, MYC-negative lymphoma, EBV-positive lymphoma, or EBV-negative lymphoma. When directed at MYC-positive lymphoma or EBV-positive lymphoma, BCV, a pharmaceutically acceptable salt thereof, or a solvate thereof may exert a strong therapeutic effect. When directed at MYC-positive lymphoma, BCV, a pharmaceutically acceptable salt thereof, or a solvate thereof may exert a strong therapeutic effect on lymphoma having either EBV positivity or EBV negativity and lymphoma having both.

In (1) to (5) above, "positivity" includes positive gene expression. Positivity may include an increase in gene expression compared to that in a healthy individual or an individual previously confirmed to be negative. In (1) to (5) above, the detection of the expression level of MYC or EBV or the positivity or negativity for MYC or EBV in a subject or a subject's lymphoma can be performed, for example, by collecting a sample from the subject (e.g., lymphoma cells, plasma, whole blood, or tissue) and measuring the gene expression of MYC or EBV in the sample. Gene expression can be measured, for example, using RT-PCR, a DNA chip, or immunostaining. In this case, a sample collected from a subject may be compared with a sample collected from a healthy individual or an individual previously confirmed to be negative. When the gene expression of MYC or EBV is significantly enhanced in the sample collected from the subject, the subject may be considered to be MYC-positive or EBV-positive. The gene expression of EBV may be examined using the expression of an EBV-derived transcription product (e.g., EBER, EBNA1, or the like) as an index. Furthermore, the detection of the expression level of MYC or EBV or the positivity or negativity for MYC or EBV in a subject or a subject's lymphoma can be performed, for example, by collecting a lymph node from the subject and subjecting it to immunostaining for MYC or EBV. In this case, the sample collected from the subject may be compared with a sample collected from a healthy individual or an individual previously confirmed to be negative. When the staining intensity for MYC or EBV is significantly enhanced in the sample collected from the subject, the subject may be considered to be MYC-positive or EBV-positive. The enhancement of the gene expression or the staining intensity as described above includes, for example, an increase by a factor of 1.5, 2, 3, 4, 5, 10, 20, or 50 or more, or by a factor within the range between any two of these values, relative to a reference subject. Furthermore, in the detection of the positivity or negativity for MYC or EBV in a subject or a subject's lymphoma, the subject may be considered to be MYC-positive or EBV-positive, for example, when the percentage of cells immunostained for MYC or EBV in the sample is greater than a predetermined percentage. This percentage may be, for example, 10, 20, 30, 40, 50, 60, 70, 80, or 90% or more, or 100%, or may fall within the range between any two of these values. When using the expression level of EBV or MYC as an index, "a high expression level of EBV or MYC in a sample from a subject" may include, for example, a level higher than the expression level of EBV or MYC in a reference subject. This higher level may be, for example, a level increased by a factor of 1.5, 2, 3, 4, 5, 10, 20, or 50 or more, or by a factor within the range between any two of these values, relative to a reference subject. The reference subject may be a sample that was previously confirmed not to have high expression of EBV or MYC, or an EBV-negative or MYC-negative sample. Furthermore, for example, "the expression level of EBV or MYC in a sample from a subject is high" may include a state in which the expression level is relatively high among a group of samples derived from subjects having lymphoma. The sample group may include, for example, a sample derived from a general patient having lymphoma. The evaluation of high expression or positivity may be conducted based on the common general technical knowledge of those skilled in the art, and, for example, may be carried out using one of the methods described in this paragraph. Furthermore, the detection of the expression level of MYC or the positivity or negativity for MYC in a subject or a subject's lymphoma can be performed, for example, by detecting MYC translocation via fluorescence in situ hybridization (FISH) (see, e.g., Salam et al., J Cancer 2020; 11(1): 190-198 or Epperla et al., Cancer. 2017 Nov 15; 123(22): 4411-4418). Furthermore, the detection of the expression level of EBV or the positivity or negativity for EBV in a subject or a subject's lymphoma can be performed, for example, by quantifying EBV DNA in plasma or whole blood using a commercially available DNA quantification kit (e.g., AccuGene m-EBV (Abbott Japan LLC) or by detecting IgM antibodies against EBV capsid antigens in serum using a commercially available antibody detection kit (e.g., BioPlex EBV IgM Kit (Bio-Rad Laboratories, Inc.)). The detection of the expression level of MYC or EBV or the positivity or negativity for MYC or EBV in a subject or a subject's lymphoma may be performed, for example, in vitro, ex vivo, or in vivo.

In (1) to (5) above, examples of lymphomas include a double-hit lymphoma and a triple-hit lymphoma. Examples of double-hit lymphomas include a lymphoma with a gene rearrangement of MYC and BCL2. Examples of triple-hit lymphomas include a lymphoma with a gene rearrangement of MYC, BCL2, and BCL6. The detection of a double-hit lymphoma or a triple-hit lymphoma can be performed, for example, by detecting translocation via FISH (see, e.g., Salam et al. (supra) or Epperla et al. (supra)). Examples of lymphomas include a high-grade lymphoma.

In (1) to (5) above, MYC includes a protein known as a transcriptional regulator. In this specification, MYC, Myc, and c-Myc can be used interchangeably or with substantially the same intent. The primary accession number for MYC described in UniProt is, for example, P01106. EBV is an abbreviation for Epstein-Barr virus. EBV is known as a virus having double-stranded DNA (see, e.g., Rivailler et al., J Virol. 2002 Dec; 76(23): 12055-12068 or Correia et al., J Virol. 2018 Nov 15; 92(22): e01132-18).

In (1) to (5) above, examples of chemotherapeutic agents include, but are not particularly limited to, an anticancer agent. Examples of anticancer agents include, a microtubule inhibitor, a DNA synthesis inhibitor, a growth factor inhibitor, a tyrosine kinase inhibitor, a cytotoxic substance, an immune checkpoint inhibitor, or other anticancer agents. Examples of microtubule inhibitors include a vinca alkaloid drug or a taxane drug. Examples of vinca alkaloid drugs include vincristine, vinblastine, vindesine, vinorelbine, or eribulin. Examples of taxane drugs include paclitaxel or docetaxel. Examples of DNA synthesis inhibitors include an antimetabolite, a topoisomerase inhibitor, a platinum-based formulation, an antitumor antibiotic, or an alkylating agent. Examples of antimetabolites include pemetrexed, 5-fluorouracil, S-1, gemcitabine, or capecitabine. Examples of topoisomerase inhibitors include irinotecan, nogitecan, etoposide, or sobuzoxane. Examples of platinum-based formulations include cisplatin, oxaliplatin, nedaplatin, or carboplatin. Examples of antitumor antibiotics include anthracycline drugs (e.g., doxorubicin, liposomal doxorubicin, daunorubicin, epirubicin, idarubicin, aclarubicin, amrubicin, mitoxantrone, or pirarubicin), mitomycin C, actinomycin D, bleomycin, peplomycin, or zinostatin stimalamer. Examples of alkylating agents include bendamustine, cyclophosphamide, dacarbazine, or ifosfamide. Examples of growth factor inhibitors include an EGF inhibitor, a VEGF inhibitor, an FGF inhibitor, or IGF inhibitor. Examples of growth factor inhibitors include bevacizumab, cetuximab, or panitumumab. Examples of tyrosine kinase inhibitors include gefitinib or erlotinib. Examples of cytotoxic substances include saporin, emtansine, deruxtecan, or vedotin. Examples of immune checkpoint inhibitors include a drug that binds to an immune checkpoint molecule or its ligand to inhibit the transmission of the immunosuppressive signal, thereby releasing the suppression of T-cell activation by the immune checkpoint molecule. Examples of immune checkpoint inhibitors include an anti-CTLA-4 antibody (e.g., ipilimumab), an anti-PD-1 antibody (e.g., nivolumab or pembrolizumab), or an anti-PD-L1 antibody (e.g., atezolizumab or avelumab). Examples of other anticancer agents include L-asparaginase. Examples of the form of chemotherapeutic agents include a low molecular weight compound or a high molecular weight compound. Examples of chemotherapeutic agents include a salt (including salts listed below) of any one or more of the compounds described herein.

In (1) to (5) above, examples of salts include, but are not particularly limited to, an inorganic salt or an organic salt (see, e.g., Bharate et al., Drug Discov Today. 2021 Feb; 26(2): 384-398. or Berge et al., J Pharm Sci. 1977 Jan; 66(1): 1-19.). Examples of salts include a metal salt, an ammonium salt, a salt with an organic base, a salt with an inorganic acid, a salt with an organic acid, or a salt with a basic or acidic amino acid. Examples of metal salts include an alkali metal salt (e.g., a sodium salt, a potassium salt, or the like), an alkaline earth metal salt (e.g., a calcium salt, a magnesium salt, a barium salt, or the like), or an aluminum salt. Examples of salts with organic bases include salts with organic bases such as trimethylamine, triethylamine, pyridine, picoline, 2,6-lutidine, ethanolamine, diethanolamine, triethanolamine, cyclohexylamine, dicyclohexylamine, or N, N'-dibenzylethylenediamine. Examples of salts with inorganic acids include salts with inorganic acids such as hydrochloric acid, hydrobromic acid, nitric acid, sulfuric acid, or phosphoric acid. Examples of salts with organic acids include salts with organic acids such as formic acid, acetic acid, trifluoroacetic acid, phthalic acid, fumaric acid, mesylic acid, tosylic acid, oxalic acid, tartaric acid, maleic acid, citric acid, succinic acid, malic acid, methanesulfonic acid, benzenesulfonic acid, or p-toluenesulfonic acid. Examples of salts with basic amino acids include salts with basic amino acids such as arginine, lysine, or ornithine. Examples of salts with acidic amino acids include salts with acidic amino acids such as aspartic acid or glutamic acid. Examples of salts include a pharmaceutically acceptable salt. In one embodiment of the present invention, the term "pharmaceutically acceptable" encompasses a form that has a reasonable benefit for pharmaceutical use. In one embodiment of the present invention, one form of a compound or a salt thereof includes a solvate form thereof. In (1) to (5) above, a solvate includes a form of a compound formed by a solute and a solvent (e.g., see Healy et al., Adv Drug Deliv Rev. 2017 Aug 1; 117: 25-46.). Examples of solvate include, but are not particularly limited to, a hydrate (e.g., a monohydrate, a dihydrate, a trihydrate, or the like) or a solvate with an organic solvent (e.g., a solvate with alcohol (methanol, ethanol, propanol, or the like), acetone, dimethylformamide, or ethyl acetate). Thesolvent includes a solvent that can substantially maintain the physiological activity of a solute after forming a solvate. The solvate includes a pharmaceutically acceptable solvate.

In (1) to (5) above, the treatment includes the ability to exert a symptom-improving effect, a suppressive effect, a recurrence-suppressing effect, or a preventive effect on a patient's disease or one or more symptoms associated with the disease. Examples of treatments include treatment that suppresses the proliferation of patient's lymphoma cells, treatment that reduces a lymphoma marker, treatment that suppresses lymph node swelling, or treatment that suppresses lymphoma recurrence. In (1) to (5) above, the pharmaceutical composition may be produced by any method known in the technical field of pharmaceutics, wherein, for example, an active ingredient and one or more pharmaceutically acceptable carriers are mixed. Furthermore, the pharmaceutical composition may be used in any form without limitation as long as it is intended for treatment, and may consist of only the active ingredient or a mixture of the active ingredient and optional ingredient(s). The form of the above-described carrier is not particularly limited and may be, for example, a solid or a liquid (e.g., a buffer). The content of the above-described carrier may be, for example, a pharmaceutically effective amount. The effective amount may be, for example, an amount sufficient to pharmaceutically stabilize or deliver the active ingredient. For example, the buffer is effective in stabilizing the active ingredient in a vial. The pharmaceutical composition may also contain a stabilizer (e.g., mannitol), a buffer (e.g., arginine), or a pH modifier (e.g., NaOH). The dosage, dosing interval, administration method, and administration route are not particularly limited and may be selected as appropriate based on factors such as the patient's age, body weight, symptoms, or the organ to be treated. The pharmaceutical composition preferably contains an active ingredient in a therapeutically effective amount or an amount effective to exert the desired action. **In** one embodiment of the present invention, the therapeutically effective amount includes an amount necessary for achieving a clinically observable improvement of symptoms in a patient. In one embodiment of the present invention, "pharmaceutically acceptable" includes a state suitable for use that is within the scope of sound medical judgment and is commensurate with a reasonable benefit/risk ratio. Ingredients other than BCV in the pharmaceutical composition are not particularly limited as long as they do not impair the effects of the present invention and may be selected as appropriate depending on the purpose. In (1) to (5) above, for example, the treatment may not include a combined use with probenecid, a uricosuric agent, or a drug for reducing side effects, if specifically indicated. Examples of side effects include diarrhea or nephrotoxicity.

In (1) to (5) above, the administration route of BCV, a pharmaceutically acceptable salt thereof, or a solvate thereof, or a pharmaceutical composition thereof to a subject is preferably one that is effective for treatment, and may be, for example, oral, intravenous, subcutaneous, intramuscular, intraperitoneal routes, or the like. The dosage form is preferably one that is effective for treatment and may be, for example, a solid formulation (e.g., a tablet), a liquid formulation (e.g., an oral suspension), or an injectable formulation (e.g., an intravenous injection).

In (1) to (5) above, the dosage, dosing interval, and administration method of BCV, a pharmaceutically acceptable salt thereof, or a solvate thereof, or a pharmaceutical composition thereof to a subject may be selected as appropriate based on factors such as the patient's age, body weight, symptoms, or the organ to be treated. The dosage may be, for example, 0.01 to 200 mg/kg body weight per administration. The dosing interval may be, for example, once or twice every 1 to 28 days or every 1 to 4 weeks. More specifically, the administration includes intravenous injection at a dosage of 20 to 80 mg/day. The administration may also include intravenous injection at a dosage of 20 to 80 mg/day, twice a week. **In** the combination treatment of BCV and a chemotherapeutic agent (e.g., gemcitabine, etoposide, or an immune checkpoint inhibitor), the dosage of BCV may include intravenous injection at a dosage of 10 to 40 mg/day or 10 to 20 mg/day. The administration may also include intravenous injection at a dosage of 10 to 40 mg/day or 10 to 20 mg/day, twice a week. The dosage for human administration is preferably 10 to 80 mg/day, twice a week, and the administration route is preferably intravenous injection. **In** this case, side effects can be minimized while a good therapeutic effect on lymphoma is maintained. The range of 10 to 80 mg described in this paragraph may refer to, for example, 10, 20, 30, 40, 50, 60, 70, or 80 mg, or may refer to any subrange between any two of these values.

In one embodiment of the present invention, the term "significantly" may refer to, for example, a state in which a statistical significance is evaluated using Student's t-test (one-tailed or two-tailed) and the calculated p-value is less than 0.05 or 0.01. Alternatively, the term may refer to a state in which a substantial difference exists.

All publications cited herein are incorporated by reference in their entirety. As used herein, the term "or" is used to indicate that "at least one or more" of the listed items in the text may be selected. The same applies to "or." When a "range between two values" is explicitly stated herein, the range includes the two values inclusive. As used herein, "A to B" includes A and B. As used herein, the term "having" includes suffering from a disease, in the context of disease.

While embodiments of the present invention have been described above, these are illustrative embodiments that may be included in the present invention. The present invention is not limited to these and may also adopt various configurations other than those described above. The present invention may also adopt the respective configurations or features described in the above-described embodiments in combination or independently.

### Examples

Hereinbelow, the present invention will be described in more detail with reference to Examples. However, the present invention is not limited to them.

### Example 1: Suppression of lymphoma cell proliferation

Cell viability was determined using Promega CellTiter-Glo (registered trademark) 2.0 Cell Viability Assay (Promega Corporation, Madison, Wisconsin, USA) according to the manufacturer's protocol. Specifically, lymphoma cells were plated in a 96-well plate at a concentration of 2 × 10³ cells per 100 µL of culture medium, and a drug (BCV) was added to each well at each concentration. At each time point, Promega CellTiter-Glo (registered trademark) 2.0 Cell Viability Assay reagent was added to the wells and incubated at room temperature for 10 minutes. Subsequently, absorbance at 450 nm was measured using a Tecan M200 Infinite 96-well plate reader and IControl software 1.6 (Tecan, Männedorf, Switzerland). Cell viability was evaluated as a percentage of a control absorbance. A dose-response curve was generated by plotting the percentage of viable cells against the drug concentration to analyze the growth-inhibitory effect. The IC50 values were estimated using GraphPad Prism version 8.0.2 (GraphPad Software). All reactions were performed in triplicate (n = 3). Furthermore, EBV positivity or EBV negativity of the cells was detected based on the presence or absence of an EBV-derived transcript (e.g., EBER, EBNA1, and the like).

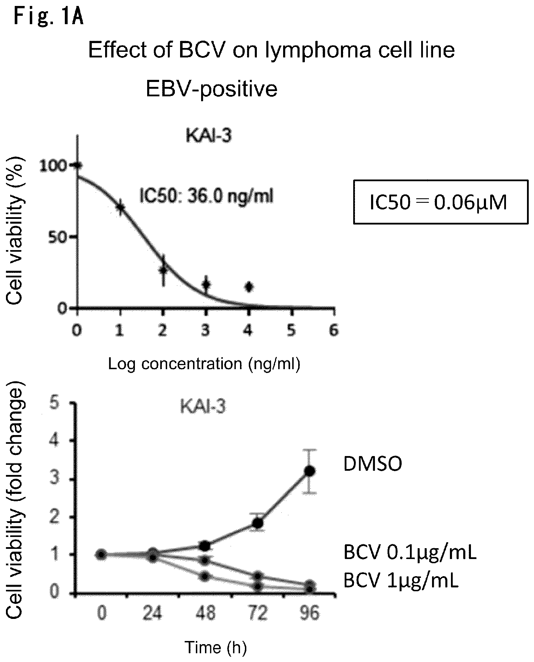

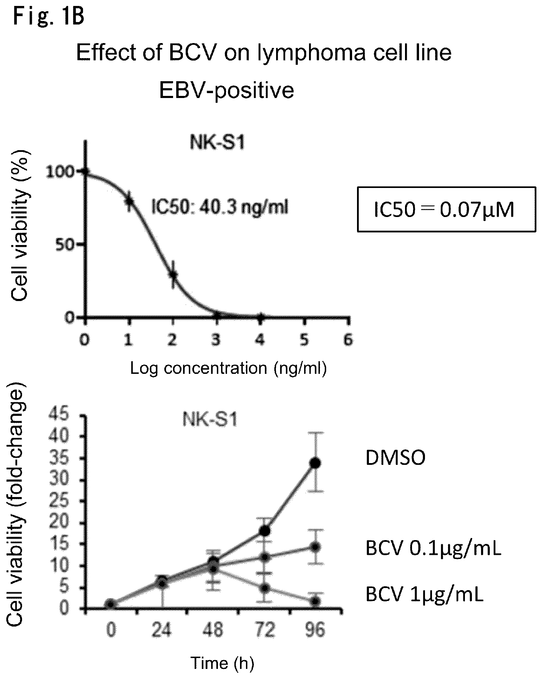

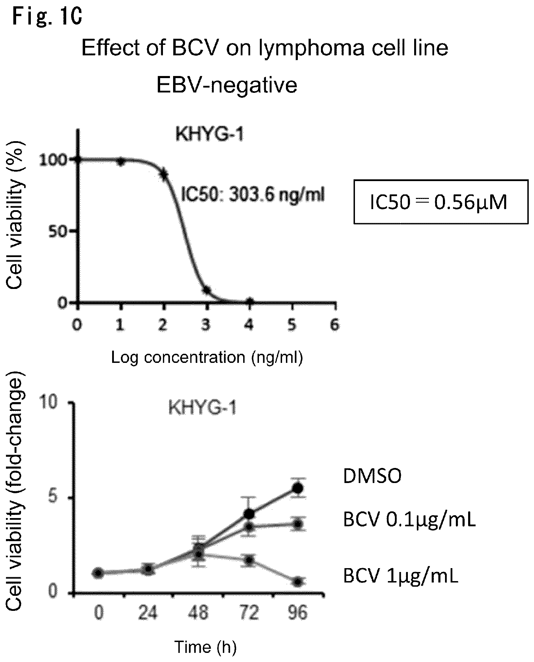

The results are shown in Fig. 1. BCV suppressed the proliferation of NK/T lymphoma cells. Regarding this cell growth-suppressive action, the IC50 value was significantly lower for EBV-positive NK/T lymphoma cells (KAI-3 and NK-S1) compared to EBV-negative NK/T lymphoma cells (KHYG-1).

### Example 2: Comparison with antiviral agents

The experimental procedure was the same as in Example 1, except that BCV, acyclovir, ganciclovir, adefovir, foscarnet, and penciclovir were used as the drugs.

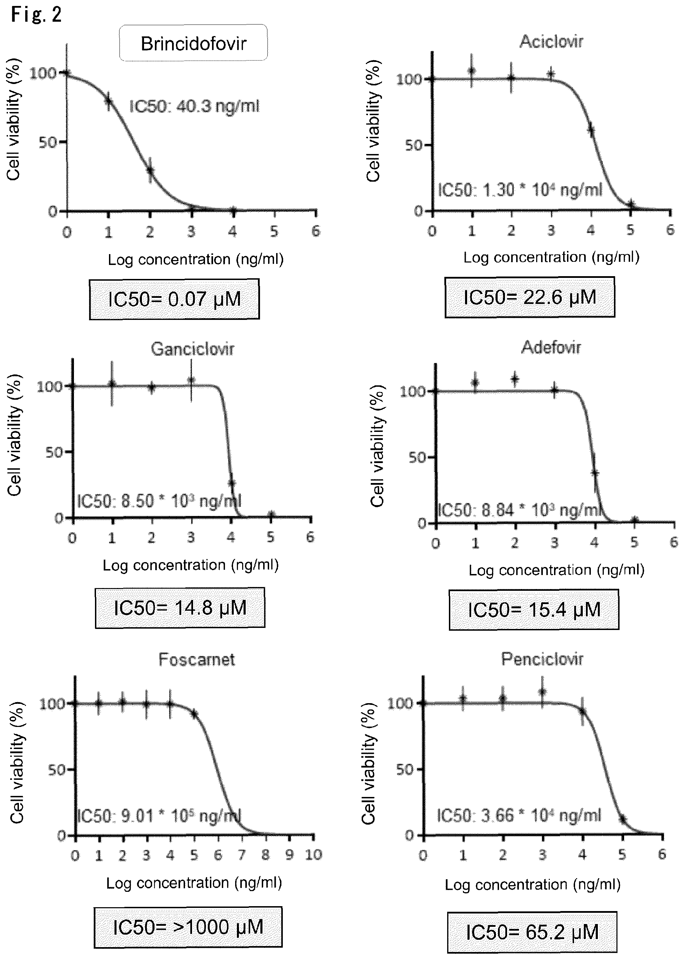

The results are shown in Fig. 2. BCV exhibited a significantly better cell growth-suppressive effect on EBV-positive NK/T lymphoma cells (NK-S1) compared to other antiviral agents (acyclovir, ganciclovir, adefovir, foscarnet, and penciclovir).

### Example 3: In vivo drug treatment

In in vivo drug treatment with BCV, 6-week-old female NSG mice were inoculated with 0.5 × 10⁶ cells (NK-S1), and BCV or a control solvent was administered intraperitoneally at a dosage of 40 mg/kg twice weekly. Tumor measurements were recorded twice weekly until the tumor size in the control group reached approximately 2000 mm³. Mice were euthanized according to the IACUC guideline. The tumor sizes of the experimental group and the control group (n = 8 per group) were averaged at each time point (Days 1, 5, 8, 12, and 15) and compared statistically. Throughout the experiment, signs of toxicity including diarrhea and weight loss were monitored.

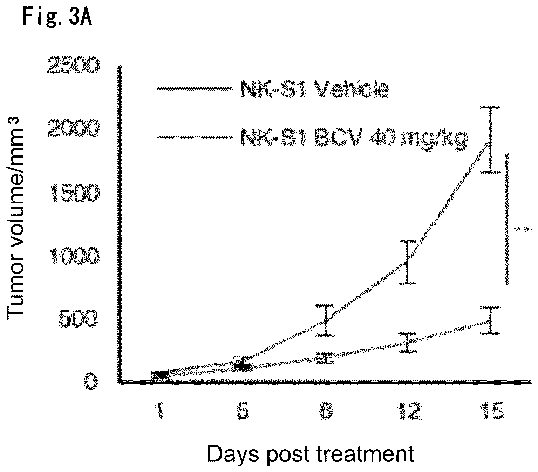

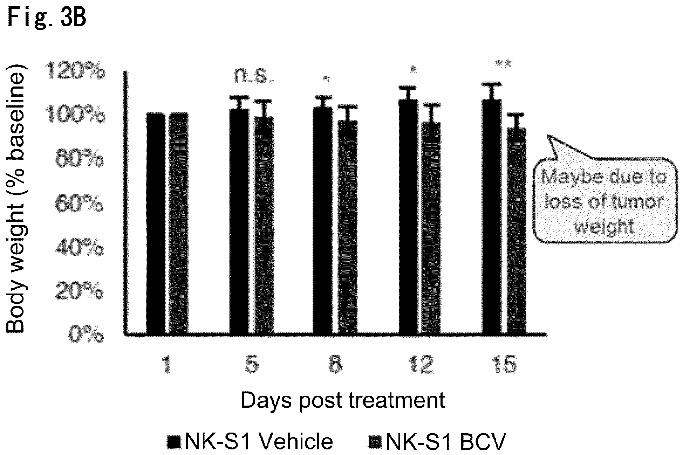

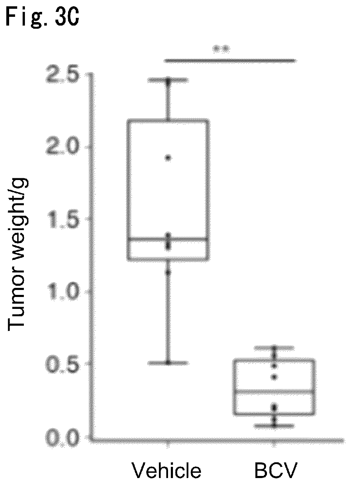

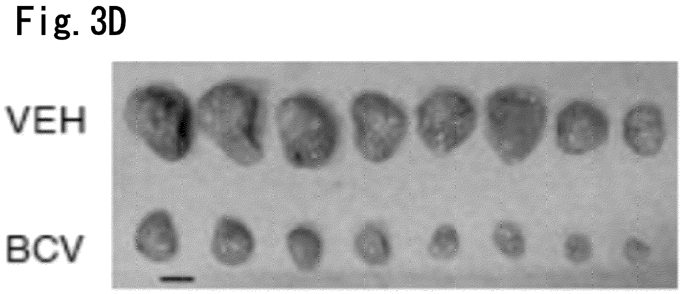

The results are shown in Figs. 3A to 3D. Administration of BCV significantly suppressed the enlargement of EBV-positive NK/T lymphoma. BCV was shown to exhibit a strong therapeutic effect in vivo. In addition, the animal's health status was good, with no diarrhea observed. This suggests that the management of side effects can be omitted or simplified.

### Example 4: Reduction in EBNA1 and LMP1 expression

NK-S1 and KAI-3 cell lines were treated with 0.1 and 1 µg/mL BCV for 72 hours. Subsequently, whole-cell lysates were separated by SDS-PAGE using 4 to 15% Mini-PROTEAN^{™} TGX Stain-Free^{™} Protein Gel (Bio-Rad Laboratories, Inc., Hercules, CA, USA) and then transferred to a 0.2 µm PVDF membrane (Bio-Rad Laboratories, Inc., Hercules, California, USA). The membrane was blocked and then gently shaken overnight at 4°C in a solution containing 5% skimmed milk powder (Bio-Rad Laboratories, Inc., Hercules, CA, USA) or 5% bovine serum albumin (Sigma-Aldrich, Darmstadt, Germany), TBST solution (50 mM Tris/HCl, pH 7.4, 150 mM NaCl, 0.1% Tween-20), and primary antibodies (anti-EBNA1 antibody (Santa Cruz Biotechnology, Inc.) and anti-LMP1 antibody (Dako)). Exposure to an appropriate HRP-conjugated anti-mouse antibody (Cytiva, Washington D.C., USA) was performed for 1 hour. Finally, chemiluminescent detection was performed using SuperSignal Substrate Western Blotting Kit (Thermo Fisher Scientific Inc., Massachusetts, USA). Imaging was performed using ChemiDoc^{™} XRS+ (a system with Image Lab^{™} software, Bio-Rad Laboratories, Inc., Hercules, California, USA).

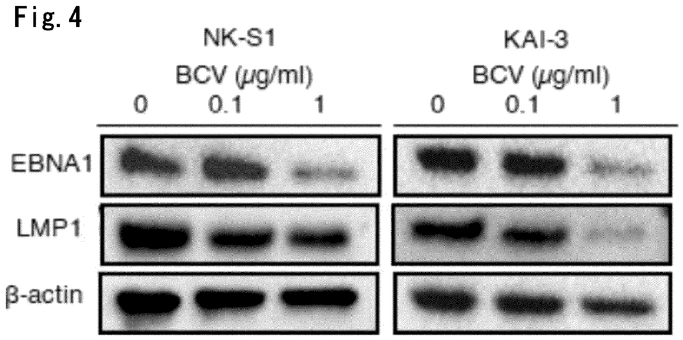

The results are shown in Fig. 4. Treatment with BCV reduced the expression level of EBNA1 and LMP1 in lymphoma cells.

### Example 5: Reduction in Myc expression

BCV-treated NK-S1 and KAI-3 cell lines were subjected to sequencing of the entire transcription product. Gene set enrichment analysis (GSEA) was performed using the Molecular Signatures Database (MSigDB) Hallmark gene set. A gene set was considered significantly enriched when the False Discovery Rate (FDR) q-value of the normalized enrichment score (NES) was less than 0.05. Quantitative PCR was performed using Maxima SYBR Green/ROX qPCR Master Mix (Thermo Scientific, USA). The ΔCt value of a gene's mRNA expression was calculated as the difference between the Ct value of the gene and that of an internal control, GAPDH. The ΔΔCt was calculated by subtracting the ΔCt value of the treatment group from the ΔCt value of an untreated control group. The fold change in expression was calculated using the formula 2^{ΔΔCt}.

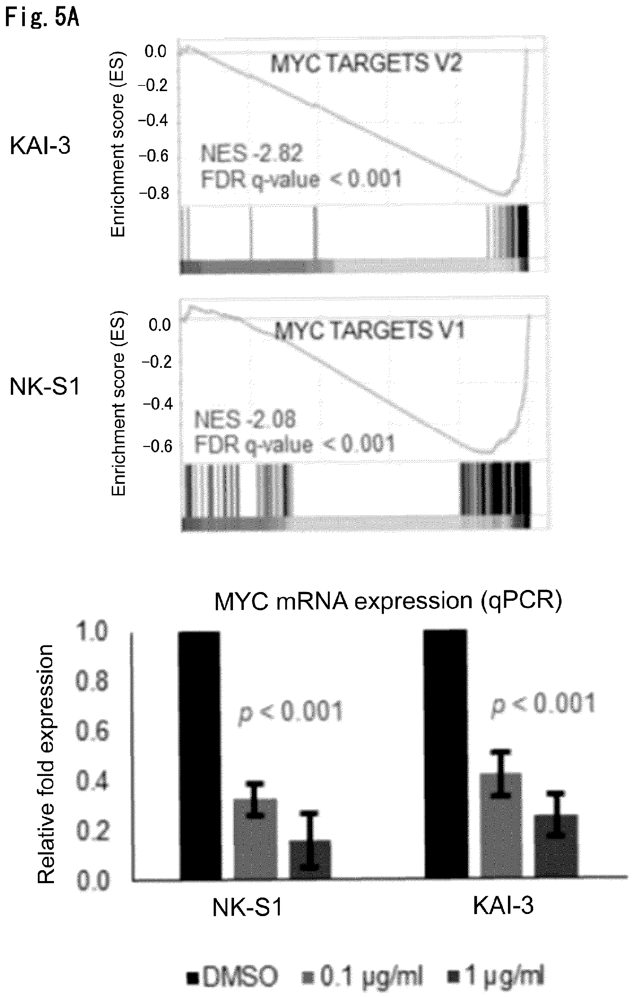

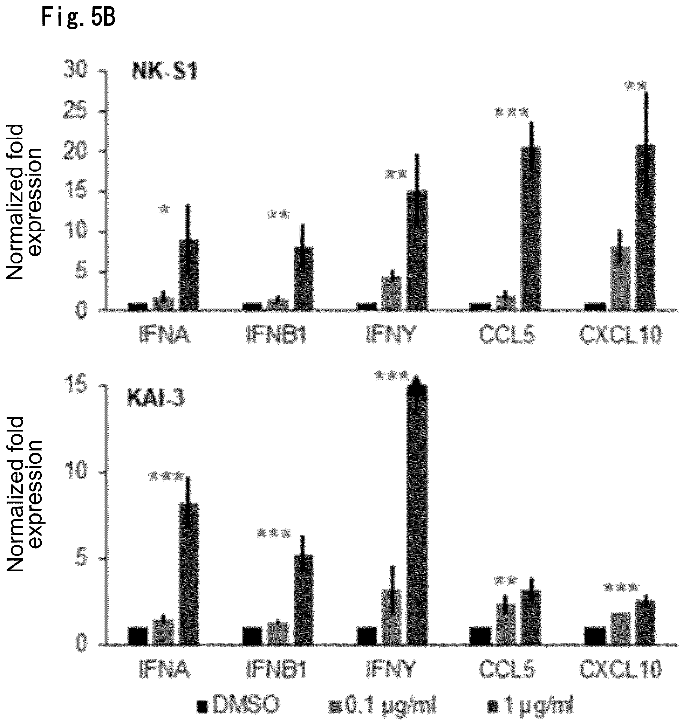

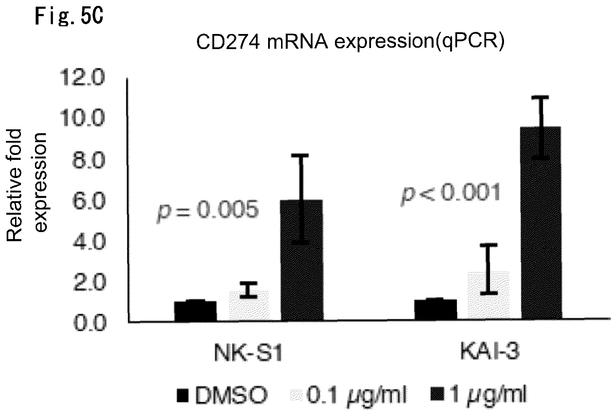

The results are shown in Figs. 5A to 5C. The action of BCV significantly reduced Myc expression and also significantly reduced the expression of a gene group controlled by MYC. On the other hand, the expression of a gene group in the STING pathway was increased.

### Example 6: Induction of immunogenic cell death

NK-S1 and KAI-3 cell lines were treated with BCV (0.1 ug/mL or 1 ug/mL) or DMSO alone for 72 hours. For calreticulin staining, cells were incubated with a calreticulin antibody (1:100, room temperature, 30 minutes) (#ab92516, Abcam Limited.) and washed prior to analysis. Then, the cells were resuspended in PBS and analyzed using a cell analyzer (BD LSR Fortessa, BD Biosciences, San Jose, CA, USA). Data were analyzed using FlowJo version 10.8.0 (BD Biosciences, San Jose, California). The extracellular level of HMGB1 in the case of BCV treatment was measured using Lumit HMGB1 immunoassay (Promega Corporation, Madison, Wisconsin, USA).

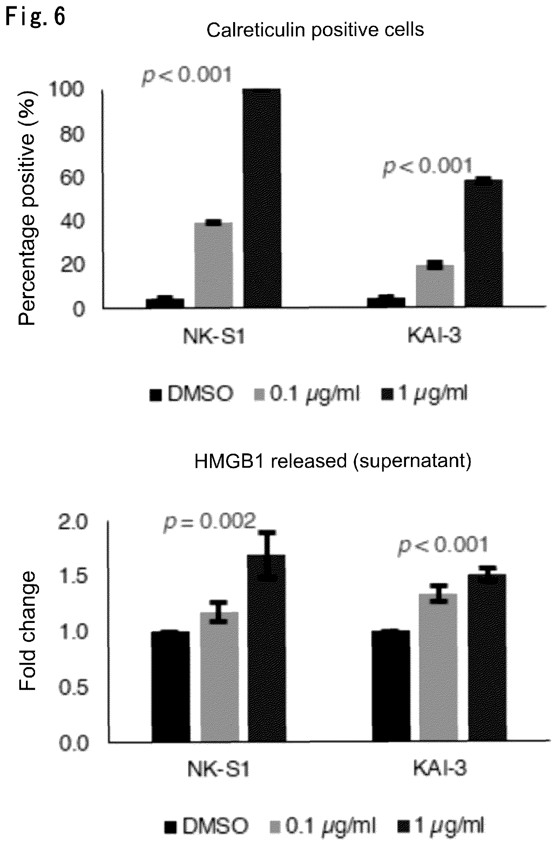

The results are shown in Fig. 6. BCV treatment clearly increased the typical phenotype reflecting immunogenic cell death, the number of calreticulin-positive cells, and the release of HMGB1.

### Example 7: Combined treatment

NK-S1 cell line was treated with a selected dose of etoposide, gemcitabine, or gamma irradiation, with or without BCV (0.1 µg/mL). Cell viability was quantified using Promega CellTiter-Glo (registered trademark) 2.0 Cell Viability Assay (Promega Corporation, Madison, Wisconsin, USA) according to the manufacturer's protocol. A combination index (CI) was calculated using Chou-Talalay median-effect analysis to determine if the effect was synergism, an additive effect, or antagonism. Specifically, a CI value less than 1 indicates synergism, and a CI value more than 1 indicates antagonism. A CI value close to 1 (i.e., between 0.9 and 1.1) was considered indicative of an additive effect.

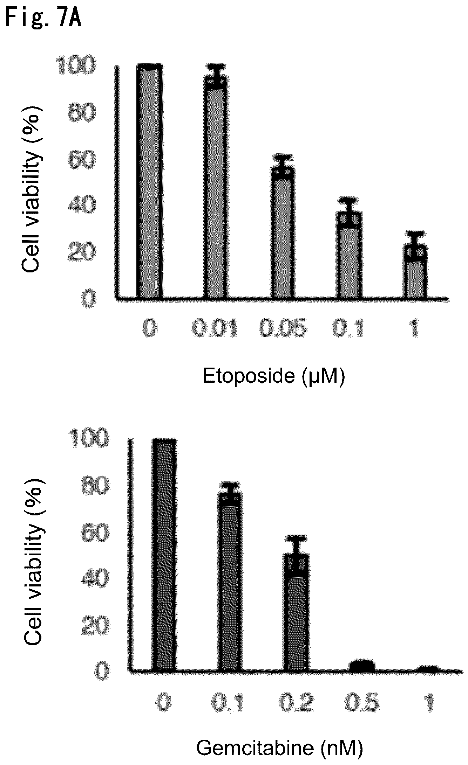

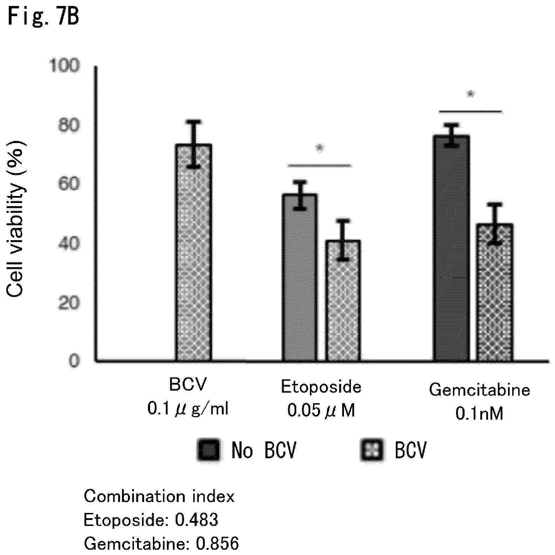

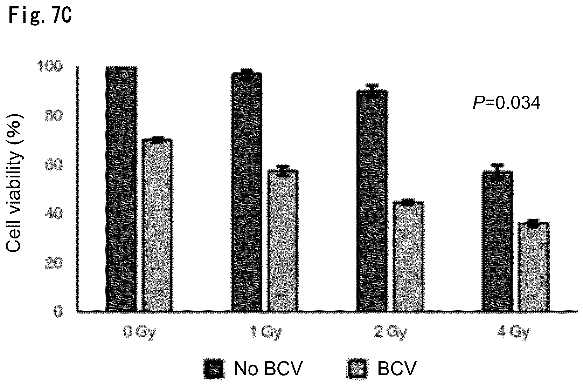

The results are shown in Figs. 7A to 7C. A significant enhancement of the effect was observed with the combined treatment of BCV and a drug, either etoposide or gemcitabine, compared to treatment with each drug alone. The effect was determined to be a synergistic effect based on the combination index value.

### Example 8: Suppression of the proliferation of MYC-positive lymphoma cells

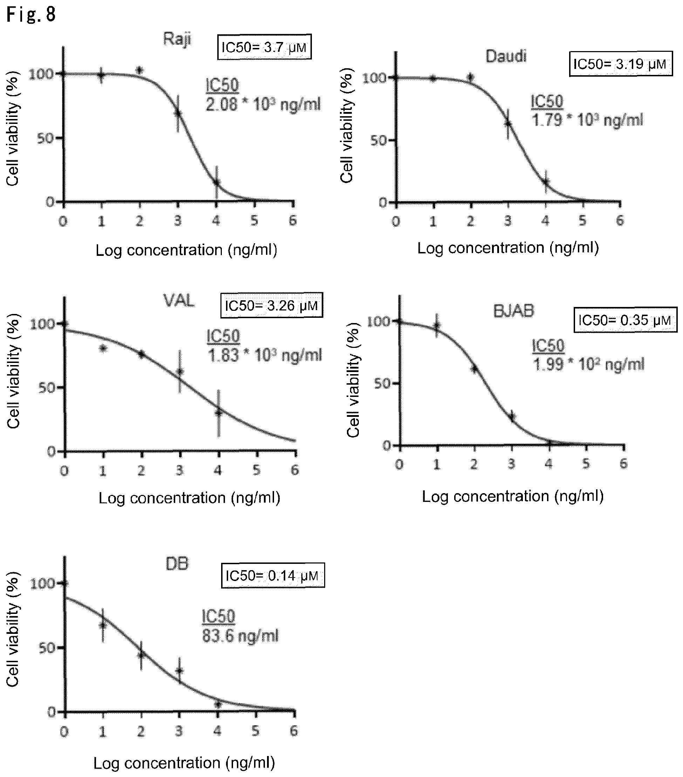

Different B-cell lymphoma cell lines were individually treated with each concentration of BCV for 96 hours. Cell viability was quantified using Promega CellTiter-Glo (registered trademark) 2.0 Cell Viability Assay (Promega Corporation, Madison, Wisconsin, USA) according to the manufacturer's protocol. Information on the presence or absence of EBV and the molecular information were obtained by referring to a public database. The cell lines shown in Fig. 8 have been reported to be classified into groups as follows: Raji and Daudi as Burkitt's lymphoma (BL) cell lines; VAL and DB as diffuse large B-cell lymphoma (DLBCL) cell lines; and BJAB as a BL cell line or a DLBCL cell line. All of these are B lymphomas in which MYC is either translocated or amplified. Raji, Daudi, and VAL are EBV-positive cell lines, while BJAB and DB are EBV-negative cell lines. VAL is triple-hit lymphoma and DB is double-hit lymphoma.

The results are shown in Fig. 8. BCV exhibited a distinct cell growth-suppressive action on MYC-positive lymphoma cells; and it exhibited a strong growth-suppressive effect on both EBV-positive cells and EBV-negative cells. In Example 5, BCV reduced Myc expression. In Example 8, BCV suppressed the proliferation of MYC-positive lymphoma cells. These results indicate that BCV is particularly suitable for the treatment of MYC-positive lymphoma.

### Example 9: Suppression of the proliferation of eleven lymphoma cells

The effect of BCV on cell viability was examined in eleven NK/T lymphoma cell lines (KAI-3, NK-S1, NK-92, KHYG-1, NK-YS, MEC-04, SNK-1, SNK-6, YT, HANK-1, or SNT-8). Cell viability was quantified using Promega CellTiter-Glo (registered trademark) 2.0 Cell Viability Assay (Promega Corporation, Madison, Wisconsin, USA) according to the manufacturer's protocol. In brief, cells were plated in a 96-well plate at a concentration of 2 × 10³ cells per 100 µL of culture medium, and a drug was added at each concentration. After each time point, Promega CellTiter-Glo (registered trademark) 2.0 Cell Viability Assay reagent was added to the wells and incubated at room temperature for 10 minutes. Subsequently, absorbance at 480 nm was measured using a Tecan M200 Infinite 96-well plate reader and iControl software 1.6 (Tecan, Männedorf, Switzerland). Cell viability was calculated as a percentage of the control absorbance. A dose-response curve was generated by plotting the percentage of viable cells against the drug concentration to analyze the growth-inhibitory effect. The IC50 values were estimated using GraphPad Prism version 8.0.2 (GraphPad Software). All reactions were performed in triplicate (n = 3).

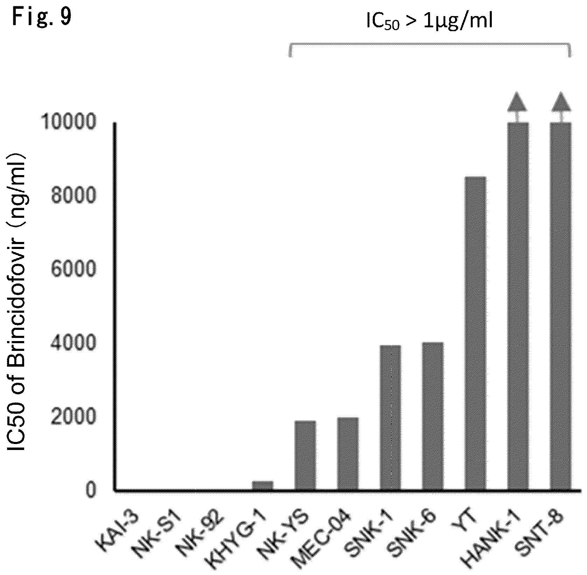

The results are shown in Fig. 9. Four cell lines (KAI-3, NK-S1, NK-92, KHYG-1) showed lower IC50 values compared to other cell lines, indicating high sensitivity to BCV.

### Example 10: Whole transcriptome sequencing of eleven lymphoma cells

Whole transcriptome sequencing was performed on the same eleven NK/T lymphoma cell lines as in Example 9. Gene set enrichment analysis (GSEA) was performed using the Molecular Signatures Database (MSigDB) Hallmark gene set to compare the top four most BCV-sensitive cell lines (KAI-3, NK-S1, NK-92, and KHYG-1) with the other seven cell lines. A gene set was considered significantly varied when the False Discovery Rate (FDR) q-value of the normalized enrichment score (NES) was less than 0.05.

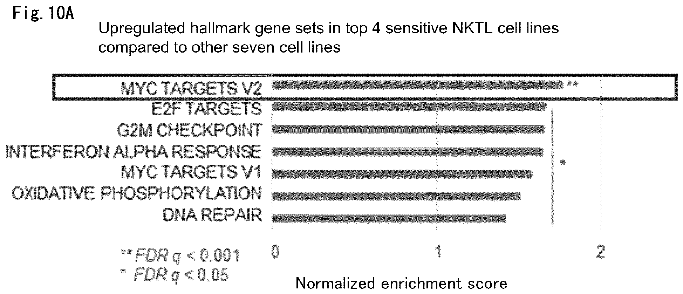

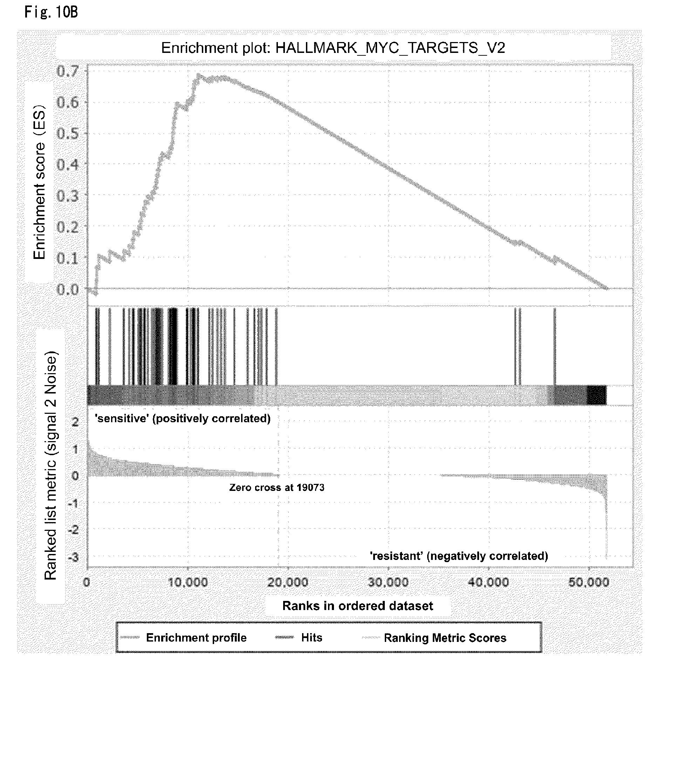

The results are shown in Figs. 10A and 10B. MYC TARGETS V2, which showed a significant increase in the figures, is a gene group regulated by MYC. As a result of the analysis, a gene group that was distinctively highly expressed in the four sensitive cell lines (KAI-3, NK-S1, NK-92, KHYG-1) compared to the other seven cell lines was found. Especially, the gene group regulated by MYC was significantly highly expressed. Thus, it was also indicated that higher MYC expression correlates with a greater antitumor effect of BCV.

### Example 11: Influence on prognosis

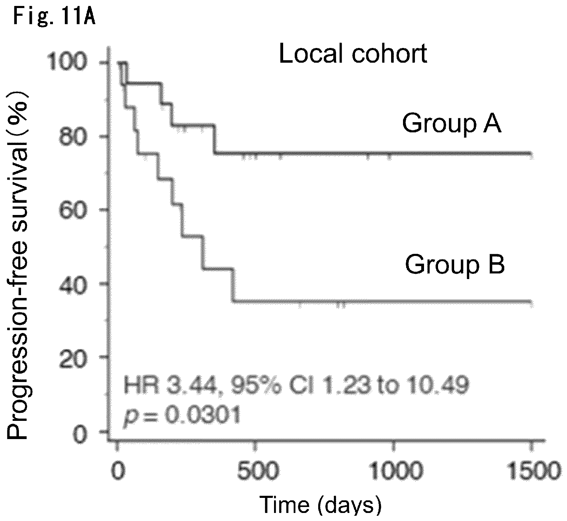

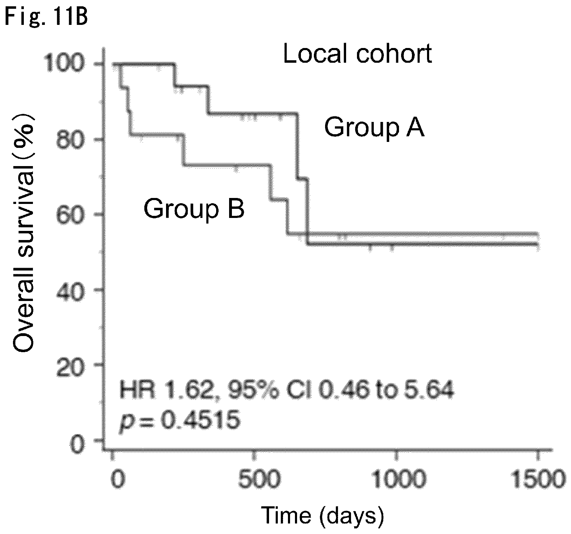

Progression-free survival and overall survival were examined in a cohort of human patients with NK/T-cell lymphoma (n = 36). Furthermore, the patient cohort was divided into group A (n = 19) and group B (n = 17) based on the gene analysis results. The enrolled patients are patients that had not received BCV treatment. The results are shown in Figs. 11A and 11B. When Group A and Group B were compared, Group A tended to have a favorable prognosis, while Group B tended to have a poor prognosis. Statistical analysis was performed using MedCalc for Windows, version 18.2.1 (MedCalc Software Ltd).

Furthermore, based on the results obtained from whole-transcriptome sequencing performed on the above-described human patient cohort (n = 36), gene set enrichment analysis (GSEA) was performed. This analysis was performed using the Molecular Signatures Database (MSigDB) Hallmark gene set to compare the favorable prognosis group and the poor prognosis group. A gene set was considered significantly varied when the False Discovery Rate (FDR) q-value of the normalized enrichment score (NES) was less than 0.05.

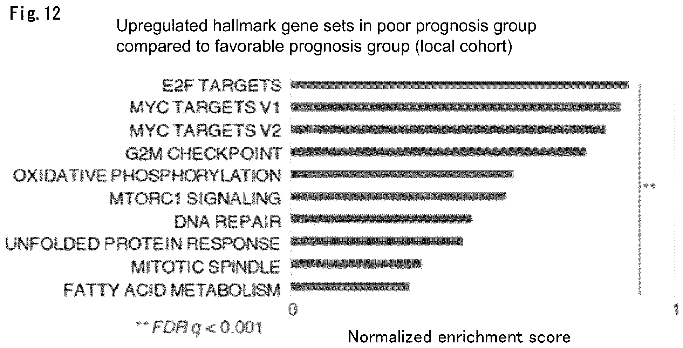

The results are shown in Fig. 12. MYC TARGETS V1 and MYC TARGETS V2, which showed a significant increase in the figures, are gene groups regulated by MYC. As a result of the analysis, it was found that the gene group regulated by MYC was significantly more highly expressed in the poor prognosis group than in the favorable prognosis group. As described in Example 5, BCV significantly reduces Myc expression and also significantly reduces the expression of the gene group controlled by MYC. Therefore, BCV administration is believed to have the effect of improving the prognosis.

Hereinabove, the present invention has been described based on the Examples. It will be understood by those skilled in the art that the Examples are for illustrative purposes only and that various modifications are possible and such modifications are also within the scope of the present invention.

## Claims

1. A pharmaceutical composition for treatment of lymphoma, comprising brincidofovir, a pharmaceutically acceptable salt thereof, or a solvate thereof.

2. The pharmaceutical composition according to claim 1, comprising brincidofovir.

3. The pharmaceutical composition according to claim 1 or 2, wherein the lymphoma is malignant lymphoma.

4. The pharmaceutical composition according to any one of claims 1 to 3, wherein the lymphoma is NK/T-cell lymphoma or B-cell lymphoma.

5. The pharmaceutical composition according to any one of claims 1 to 4, wherein the lymphoma is NK/T-cell lymphoma.

6. The pharmaceutical composition according to any one of claims 1 to 5, wherein the pharmaceutical composition reduces the MYC expression in lymphoma cells targeted by the treatment.

7. The pharmaceutical composition according to any one of claims 1 to 6, wherein the treatment comprises identifying an EBV-positive subject as a subject to be treated.

8. The pharmaceutical composition according to any one of claims 1 to 7, wherein the lymphoma is EBV-positive lymphoma.

9. The pharmaceutical composition according to any one of claims 1 to 8, wherein the treatment comprises detecting the EBV expression level in a sample from a subject.

10. The pharmaceutical composition according to any one of claims 1 to 9, wherein the treatment comprises identifying an MYC-positive subject as a subject to be treated.

11. The pharmaceutical composition according to any one of claims 1 to 10, wherein the lymphoma is MYC-positive lymphoma.

12. The pharmaceutical composition according to any one of claims 1 to 11, wherein the treatment comprises detecting the MYC expression level in a sample from a subject.

13. The pharmaceutical composition according to any one of claims 1 to 12, wherein the treatment comprises identifying an EBV-positive and MYC-positive subject as a subject to be treated.

14. The pharmaceutical composition according to any one of claims 1 to 13, wherein the lymphoma is EBV-positive and MYC-positive.

15. The pharmaceutical composition according to any one of claims 1 to 14, wherein the treatment comprises detecting the expression levels of EBV and MYC in a sample from a subject.

16. The pharmaceutical composition according to any one of claims 1 to 15, wherein the pharmaceutical composition improves prognosis.

17. The pharmaceutical composition according to any one of claims 1 to 16, wherein the pharmaceutical composition suppresses the proliferation of lymphoma cells.

18. The pharmaceutical composition according to any one of claims 1 to 17 for use in the combination treatment of brincidofovir, a pharmaceutically acceptable salt thereof, or a solvate thereof and a chemotherapeutic agent.
